# EUROPEAN PATENT APPLICATION

(11) **EP 0 634 168 A1**
(43) Date of publication of application: **18.01.1995**
(21) Application number: 94905207.0
(22) Date of filing: 26.01.1994
(51) Int. Cl.: A61K 31/195, A23L 1/305

(54) **NUTRITIVE COMPOSITION**

(30) Priority: 29.01.1993 JP 14240/93
(71) Applicant: KYOWA HAKKO KOGYO CO., LTD., Chiyoda-ku, Tokyo 100 (JP)
(72) Inventor: HARA, Takahiro, 403-1, Nishi-ku 2-chome, Ibaraki 300-11 (JP); YOKOO, Yoshiharu, 3010-34, Ushiku-cho, Ibaraki 300-12 (JP)
(74) Representative: Lambert, Hugh Richmond
(86) International application number: JP9400101
(87) International publication number: WO9416688

(57) **Abstract**

A nutrient composition such as infusions and nutrient preparations, which contains β-alanyl-L-glutamine.

## Description

The present invention relates to β-alanyl-L-glutamine-containing nutrient compositions such as infusions and nutrient preparations.

As a means for supplementing nutrients administered to mammals including humans either orally or through injection, it is known to add individual amino acids either alone or in combination with proteins, vitamins and the like. For example, nutrients given to a patient in post-operative catabolic condition must be supplemented with L-glutamine since the quantity of L-glutamine in the muscle of such a patient is seriously decreased. However, because of its heat instability, L-glutamine itself cannot be added to a nutrient composition which requires heat sterilisation prior to use.

Against that, it is known to supply L-glutamine to nutrient compositions in the form of a dipeptide, for example, as L-alanyl-L-glutamine (JPA-61-247354), L-aspartyl-L-glutamine (JPA-62-151156) or as L-glutamyl-L-glutamine (WO90/11024, USP5134125). Compared with L-glutamine itself the heat stability of such dipeptides is improved but is still unsatisfactory.

An L-glutamine-containing substance of improved heat stability is therefore still much in demand.

In accordance with the present invention it has been found that β-alanyl-L-glutamine-containing nutrient compositions have that stability and an improved solubility in water.

Examples of nutrient compositions which may be supplemented with β-alanyl-L-glutamine in accordance with this invention include amino acid infusions and nutrient preparations.

Usually such amino acid infusions will contain in addition to β-alanyl-L-glutamine, at least one essential amino acid, such as glycine and L-isomers of isoleucine, leucine, lysine, phenylalanine, methionine, threonine, tryptophan, valine, arginine, histidine, alanine, aspartic acid, cysteine, glutamic acid, proline, serine and tryosine and salts of those essential amino acids. If necessary, the amino acid infusions may additionally contain carbohydrates such as glucose, fructose, xylitol, sorbitol and maltose, polyhydric alcohols such as glycerol, lipids such as soy bean oil, cotton seed oil, sesame oil, yolk lecithin and soy bean lecithin, vitamins such as vitamin A, vitamin B₁, vitamin B₂, vitamin B₆, nicotinic acid, pantothenic acid, vitamin C, vitamin D, vitamin E, biotin and folic acid, electrolytes such as sodium chloride, sodium acetate, potassium chloride, magnesium sulphate, magnesium chloride, calcium chloride, dipotassium hydrogenphosphate and sodium dihydrogenphosphate, halogens such as iodine, and metals such as iron, zinc, manganese, copper and cobalt.

The β-alanyl-L-glutamine used in the present invention is a known compound, and can be synthesized by the process described, for example, in CA, Vol. 70, 38090n (1969).

The amino acid infusions of this invention can be obtained in either of the following ways. In the first method, a parenterally administrable infusion is prepared either by adding β-alanyl-L-glutamine and at least one of the above-mentioned amino acids and additives an amount of distilled water or physiological saline solution equal to from 33 to 10,000 times the weight of the β-alanyl-L-glutamine followed by thorough stirring to completely dissolve solids.

In a second method from 0.001 to 30 wt % of β-alanyl-L-glutamine, is added to a solution of one or more of the aforementioned essential amino acids followed by dissolving.

In either method the β-alanyl-L-glutamine and amino acids can be dissolved in the solution and, if necessary, by heating the solution to 25 to 50°C.

The pH of the obtained solution may then be adjusted to 4.0 to 7.5 with acetic acid, hydrochloric acid, lactic acid, malic acid, citric acid, succinic acid, fumaric acid or sodium hydroxide as appropriate and the resulting solution then subjected to a sterilisation treatment such as heat sterilisation or sterilising filtration (ultra filtration) to obtain sterilised amino acid infusion. Generally, such amino acids infusion are administered intravenously.

A typical example of a composition according to the present invention is illustrated in Table 1.

**Table 1**

| **Amino Acid** | **(mg/dl)** |
|---|---|
| L-Isoleucine | 160 - 1600 |
| L-Leucine | 180 - 2000 |
| L-Lysine hydrochloride | 180 - 2400 |
| L-Phenylalanine | 130 - 1400 |
| L-Methionine | 50 - 1200 |
| L-Threonine | 80 - 720 |
| L-Tryptophan | 30 - 350 |
| L-Valine | 70-1600 |
| L-Arginine hydrochloride | 120 - 2500 |
| L-Histidine hydrochloride | 50 - 900 |
| Glycine | 0 - 2500 |
| L-Alanine | 0 - 2000 |
| Sodium L-Aspartate | 0 - 1300 |
| L-Cysteine | 0 - 200 |
| Sodium L-glutamate | 0 - 1300 |
| β-alanyl-L-glutamate | 1 - 20000 |
| L-Proline | 0 - 1080 |
| L-Serine | 0 - 1200 |
| L-Tyrosine | 0 - 90 |

The nutrient composition of the present invention are mainly formulated for oral or enteral administration. In the case of oral administration, other ingredients such as proteins, carbohydrates, lipids, vitamins, minerals, pseudo-tasting agents, sweeteners, flavouring agents and dyes, are preferably added.

Suitable proteins include casein, hydrolysed products thereof, gelatin, skim milk and powder of egg yolk. Suitable carbohydrates include starch, dextrin, cyclodextrin, glucose, reduced maltose, lactose and malt extracts. Suitable lipids include middle chain fatty acids and yolk oil. Suitable vitamins include vitamin A, thiamine, riboflavin, pyridoxine, niacin, pantothenic acid, cyanocobalamin, L-ascorbic acid and α-tocopherol. Suitable minerals include sodium chloride, potassium chloride, calcium chloride and iron lactate. Suitable pseudo-tasting agents include inosinic acid. Suitable sweeteners include aspartame, D-xylose and D-sorbitol. Suitable flavouring agents include citric acid and orange flavours. Suitable dyes include β-carotene and copper chlorophyll.

Suitable nutrient preparations according to the present invention can be obtained by adding to 1 litre of water from 0.02 to 500 g (0.002 to 50% by weight), preferably 1 to 300 g (0.1 to 30% by weight) protein, 0.02 to 800 g (0.002 to 80% by weight), preferably 1 to 500 g (0.1 to 50% by weight) carbohydrate, and 0.01 to 300 g (0.001 to 30% by weight), preferably 1 to 150 g (0.1 to 15% by weight) β-alanyl-L-glutamate, followed by stirring and dissolving, if necessary with heating at 40 to 120°C.

Nutrient preparations, in liquid or jelly form, can first be sealed in a waterproof bag, bottle, can, etc. and then thermally sterilised. Alternatively, the liquid nutrient preparation can be sterilised and then dried by freeze-drying or the like and stored in a dry state. In that case the freeze dried solid can be redissolved in water etc., just before using.

The heat stability and solubility of β-alanyl-L-glutamine is shown in the following test examples.

### Test Example 1

10 mM solutions of β-alanyl-L-glutamine, L-alanyl-L-glutamine (control) and L-glutamine (control) were adjusted to a pH of 6.5 with sodium hydroxide and heated in an autoclave at 120°C for periods of 20 minutes and 120 minutes. The amount of each compound remaining after heating was then determined. The results are shown in Table 2.

**Table 2**

| | **% of compound remaining** | |
|---|---|---|
| | **After 20 min.** | **After 120 min.** |
| β-Alanyl-L-glutamine | 100% | 99% |
| L-Alanyl-L-glutamine | 98% | 91% |
| L-Glutamine | 9% | 0% |

Table 2 shows that when the solutions are heated at 120°C for 20 minutes, the β-alanyl-L-glutamine is unaffected, whilst in the control samples L-alanyl-L-glutamine shows some decomposition and L-glutamine shows 91 % decomposed. At 120°C for 120 minutes, L-glutamine is completely decomposed, approximately -9% if L-alanyl-L-glutamine is decomposed, but only about 1% of β-alanyl-L-glutamine.

### Test Example 2

β-alanyl-L-glutamine, L-alanyl-L-glutamine(control) and L-glutamine (control) were each dissolved in 1 litre of water (25°C) to determine their maximum solubility.

### The results are shown in Table 3.

**Table 3**

| | **Amount dissolved in 1 litre of water (g)** |
|---|---|
| β-Alanyl-L-glutamine | 952 |
| L-Alanyl-L-glutamine | 599 |
| L-Glutamine | 43 |

Table 3 shows that β-alanyl-L-glutamine has the highest solubility. Examples of the present invention are shown below.

### Example 1

A mixture of the amino acids shown in Table 4 and 10 g of β-alanyl-L-glutamine were dissolved in IR of distilled water at 70°C. The pH of the solution was adjusted to 6.5 with sodium hydroxide solution and the solution filtered through a millepore filter (0.45µm). The filtrate was packed in glass bottles in 200 ml portions, followed by blowing with aseptic nitrogen gas for 30 seconds. After sealing, the glass bottles were sterilised by heating at 120°C for 20 minutes.

**Table 4**

| | |
|---|---|
| L-Isoleucine | 4.6 g |
| L-Leucine | 7.7 g |
| L-Lysine hydrochloride | 5.0 g |
| L-Phenylalanine | 4.3 g |
| L-Methionine | 2.1 g |
| L-Threonine | 2.9 g |
| L-Tryptophan | 1.0 g |
| L-Valine | 4.9 g |
| L-Arginine hydrochloride | 6.1 g |
| L-Histidine hydrochloride | 2.6 g |
| Glycine | 3.4 g |
| L-Alanine | 4.6 g |
| Sodium L-Aspartate | 0.3 g |
| L-Cysteine | 0.3 g |
| Sodium L-glutamate | 0.3 g |
| L-Proline | 3.9 g |
| L-Serine | 2.3 g |
| L-Tyrosine | 0.3 g |

The resulting sterile infusion was suitable for injection.

### Example 2

10 g of casein hydrolysate, 2.5 g of β-alanyl-L-glutamine, 6.3 g of cyclodextrin, 0.11 g of inosinic acid, 16 g of citric acid and 6.8 h of reduced maltose were added to 500 ml of water followed by 0.07 ml of orange flavour. The resulting solution was sterilised by heating at 120°C for 20 minutes. The resulting sterilised nutrient preparation was suitable for use as an oral nutrient supplement.

### Example 3

10 g of casein hydrolysate, 8 g of gelatin, 2.5 g of β-alanyl-L-glutamine, 20 g of dextrin and 20 g of reduced maltose were added to 300 ml of water and the mixture heated at 100°C for 30 minutes. The solution was then cooled to prepare a jelly-like nutrient preparation.

## Claims

1. A nutrient composition or nutritional supplement for mammals containing β-alanyl-L-glutamine.

2. A nutrient composition or nutritional supplement according to claim 1, containing β-alanyl-L-glutamine in an amount of 0.001 to 30% by weight.

3. A nutrient composition or nutritional supplement according to claim 1, which is an amino acid infusion, optionally containing one or more of the following: a protein, carbohydrate, lipid, vitamin, non-toxic mineral, a pseudo-tasting agent, sweetener, flavouring agent or edible dye.

4. A nutrient composition according to claim 3, wherein said amino acid infusion has the following composition:
| | |
|---|---|
| L-Isoleucine | 160 - 1600 (mg/dl) |
| L-Leucine | 180 - 2000 |
| L-Lysine hydrochloride | 180 - 2400 |
| L-Phenylalanine | 130 - 1400 |
| L-Methionine | 50 - 1200 |
| L-Threonine | 80 - 720 |
| L-Tryptophan | 30 - 350 |
| L-Valine | 70 - 1600 |
| L-Arginine hydrochloride | 120 - 2500 |
| L-Histidine hydrochloride | 50 - 900 |
| Glycine | 0 - 2500 |
| L-Alanine | 0 - 2000 |
| Sodium L-Aspartate | 0 - 1300 |
| L-Cysteine | 0 - 200 |
| Sodium L-glutamate | 0 - 1300 |
| β-alanyl-L-glutamate | 1 - 20000 |
| L-Proline | 0 - 1080 |
| L-Serine | 0 - 1200 |
| L-Tyrosine | 0 - 90 |

5. A nutrient composition according to any one of claims 1-4 which contains or additionally contains from 0.002 to 50% by weight proteins, and 0.002 to 80% by weight carbohydrate.

6. The use of β-alanyl-L-glutamine as or in a nutrient supplement for mammals.

7. The use, according to claim 6, of β-alanyl-L-glutamine as in a nutrient supplement for human patients when in a post-operative catabolic condition.

8. The use, according to claim 6 or 7, of a β-alanyl-L-glutamine composition as claimed in any one of claims 1-5.
